(19) Europäisches Patentamt
European Patent Office
Office européen des brevets

(11) EP 0 975 420 B1

(12) # EUROPÄISCHE PATENTSCHRIFT

(45) Veröffentlichungstag und Bekanntmachung des Hinweises auf die Patenterteilung:
**14.11.2001 Patentblatt 2001/46**

(21) Anmeldenummer: **98910721.4**

(22) Anmeldetag: **04.03.1998**

(51) Int Cl.⁷: **B01F 17/00**, E21B 37/06, C10G 75/04, C10L 1/14, C08L 61/06, C08K 5/13, C08L 95/00

(86) Internationale Anmeldenummer:
**PCT/EP98/01199**

(87) Internationale Veröffentlichungsnummer:
**WO 98/40158 (17.09.1998 Gazette 1998/37)**

(54) **SYNERGISTISCHE MISCHUNGEN VON ALKYLPHENOLFORMALDEHYDHARZEN MIT OXALKYLIERTEN AMINEN ALS ASPHALTEN-DISPERGATOREN**

SYNERGISTIC MIXTURES OF ALKYL PHENOL FORMALDEHYDE RESINS WITH OXALKYLATED AMINES AS ASPHALT DISPERSING AGENTS

MELANGES SYNERGIQUES DE RESINES DE FORMALDEHYDE D'AKYLPHENOL AVEC DES AMINES OXALKYLEES COMME DISPERSANTS D'ASPHALTENE

(84) Benannte Vertragsstaaten:
**DE FR GB IT NL**

(30) Priorität: **10.03.1997 DE 19709797**

(43) Veröffentlichungstag der Anmeldung:
**02.02.2000 Patentblatt 2000/05**

(73) Patentinhaber: **Clariant GmbH**
**65929 Frankfurt am Main (DE)**

(72) Erfinder:
- **MILLER, Dennis**
  **D-65779 Kelkheim (DE)**
- **FEUSTEL, Michael**
  **D-55278 Köngernheim (DE)**
- **VOLLMER, Axel**
  **D-65830 Kriftel (DE)**
- **VYBIRAL, Reinhard**
  **D-84508 Burgkirchen (DE)**
- **HOFFMANN, Dieter**
  **D-84556 Kastl (DE)**

(56) Entgegenhaltungen:
**EP-A- 0 579 339**
**EP-A- 0 584 708**
**DE-B- 1 284 378**
**US-A- 2 760 852**
**US-A- 3 390 088**
**US-A- 5 021 498**
**US-A- 5 494 607**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).



Printed by Jouve, 75001 PARIS (FR)

## Beschreibung

Asphaltene sind Bestandteile von Rohölen. Sie enthalten eine Vielzahl von Strukturen, besonders hochmolekulare kondensierte aromatische Komponenten mit Heteroatomen. Angesichts der Komplexität ihrer Chemie werden Asphaltene als die Ölfraktion beschrieben, die in Benzol, aber nicht in n-Pentan löslich ist.

Im Rohöl liegen Asphaltene normalerweise als kolloidale Dispersion vor. Diese wird durch Ölharze stabilisiert.

Asphaltene können während der Produktion, der Raffination, des Transports und der Lagerung von Rohöl und davon abgeleiteten Produkten, wie z.B. schweres Heizöl oder Schiffsöl, ausfallen. Gemeinsame Ursachen für dieses Ausfallen sind ein Absinken der Temperatur oder ein Wechsel in der Zusammensetzung (z.B. Verdampfung von leicht flüchtigen Bestandteilen). Asphaltene können auch beim Fließen durch poröse Medien ausfallen. Fluten mit $CO_2$ während des Förderprozesses kann Asphaltene zum Flokkulieren oder zum Ausfallen bringen.

Manche Öle enthalten Kohlenwasserstoffwachse, die bei niedrigen Temperaturen ausfallen. Wechselwirkungen zwischen dem Ausfallen von Wachs und Asphaltenen können die Gesamtmenge an ausgefallener Substanz oder deren Bildungsgeschwindigkeit erhöhen.

Ausgefallene Asphaltene verursachen Probleme bei der Produktion und bei der Verarbeitung von Rohölen. Asphaltene schlagen sich nieder in Ventilen, Rohren und Fördereinrichtungen. An heißen Oberflächen, wie beispielsweise Wärmetauschern, kann die Carbonisierung dieser Niederschläge ihre Entfernung sehr schwierig machen. Die Niederschläge reduzieren den Wirkungsgrad von Anlagen und können im schlimmsten Fall zu einer kompletten Blokkierung und zu einem Produktionsstop führen, was hohe Kosten verursacht.

Schweröle, die oft zum Antrieb von Schiffen verwendet werden, enthalten beträchtliche Mengen an Asphaltenen. Das Ausfallen von Asphaltenen kann sowohl zu schlechter Verbrennung als auch zu Schwierigkeiten bei der Handhabung und bei der Lagerung des Treibstoffes führen.

Bitumen, Schweröle und Rückstände werden manchmal mit Lösemittel verdünnt, um die Viskosität für den Transport zu reduzieren. Wenn dabei Asphaltene ausfallen, so ergeben sich damit Probleme bei der Handhabung.

Das Ausfallen von Asphaltenen kann durch kleine Mengen an Dispergatoren verhindert oder verringert werden. Diese Substanzen zeigen einen oder mehrere der folgenden Effekte:

a) Die Menge an Niederschlag wird reduziert;
b) der Niederschlag bildet sich langsamer;
c) der Niederschlag ist feiner verteilt; und
d) die Neigung des Niederschlages, sich auf Oberflächen abzulagern, wird reduziert.

Wenn sich bereits Niederschläge an Asphaltenen gebildet haben, können sie durch den Gebrauch von Lösemitteln entfernt werden. Die Zugabe eines Dispergators kann die Wirksamkeit dieser Lösemittel verbessern.

Eine Vielzahl von Asphalten-Dispergatoren sind bereits bekannt.
CA 2 029 465 und CA 2 075 749 beschreiben Alkylphenolformaldehydharze in Kombination mit hydrophilen-lipophilen Vinylpolymeren. Die Asphaltendispergierenden Eigenschaften von Dodecylbenzolsulfonsäure wurden beschrieben in US 4 414 035, außerdem durch D.-L. Chang und H.S. Fogler (SPE paper No. 25185, 1993) und durch M.N. Bouts et al. (J. pet. Technol. 47, 782-7,1995).

Die Herstellung von oxalkylierten Aminen und deren Verwendung als Korrosionsinhibitoren, Demulgatoren und Paraffindispergatoren wird in US 5 421 993 beschrieben.

Alkylphenolformaldehydharze können durch saure oder basische Katalyse hergestellt werden. Die sauer katalysierten Verbindungen sind vorwiegend linear (vgl. Formel (I)), während die basisch katalysierten Verbindungen einen hohen Anteil von cyclischem Material wie in Formel (II) enthalten (siehe B. Rowan in D. Karsa (Hrsg) "Industrial application of surfactants III", S. 247).

DE-B-1 284 378 offenbart die Verwendung von synergistisch wirkenden Mischungen aus Alkoxylierungsprodukten, wie z.B. oxyalkylierten Fettaminen, und Naphtholen als Dispergiermitteln für Asphaltstoffen. Die dispergierende Wirkung der Mischungen läßt sich durch Zusatz von oxyalkylierten Alkylphenol-Formaldehyd-Harzen verbessern.

Die bisher bekannten Dispergatoren können die durch das Ausfallen von Asphaltenen verursachten Probleme nur teilweise lösen. Da Öle in ihrer Zusammensetzung variieren, können einzelne Dispergatoren nur in einem beschränkten Bereich wirksam arbeiten. Manchmal haben sogar kleine Änderungen in der Ölzusammensetzung einen großen Effekt auf die Dispergiereigenschaften für Asphaltene. Deshalb sind in einigen Fällen die bekannten Dispergatoren nicht zufriedenstellend und zusätzliche Typen sind erforderlich.

Es bestand somit die Aufgabe, neue Asphalten-Dispergatoren zur Verfügung zu stellen, die die beschriebenen Nachteile der bisher bekannten Dispergatoren nicht aufweisen.

Es wurde nun gefunden, daß Kombinationen von Alkylphenolharzen mit oxalkylierten Aminen eine stärkere Asphaltendispergierung bewirken als die Einzelsubstanzen. Dieser Synergismus ist überraschend, da bei einer derartigen Kombination von sauren und basischen Verbindungen man eine Reduzierung der Wirksamkeit in Folge einer

Teilneutralisation erwarten würde.

Gegenstand der Erfindung ist somit eine synergistische Mischung aus

5 bis 95 , vorzugsweise 20 bis 80 Gew.-% einer Verbindung A der Formeln (I) oder (II)

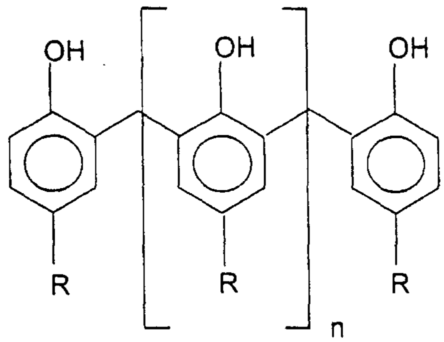

(I)

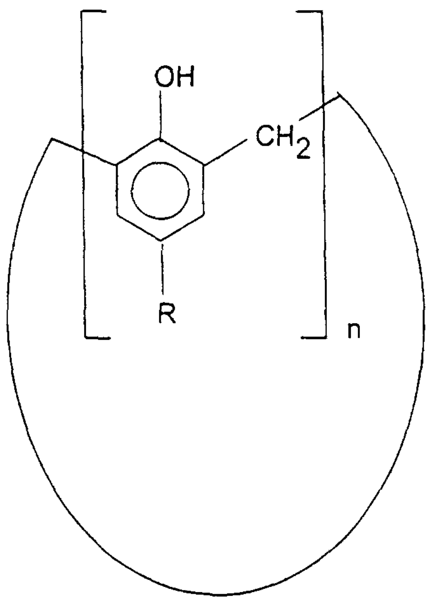

(II)

wobei

- n 2 bis 12, vorzugsweise 5 bis 9, und
- R $C_3$-$C_{24}$-, vorzugsweise $C_4$-$C_{12}$-Alkyl, insbesondere Isononyl, Isobutyl oder Amyl, $C_6$-$C_{12}$-Aryl oder -Hydroxyaryl bzw. $C_7$-$C_{12}$-Aralkyl bedeuten

und

5 bis 95, vorzugsweise 20 bis 80 Gew.-% einer Verbindung B der Formel (III)

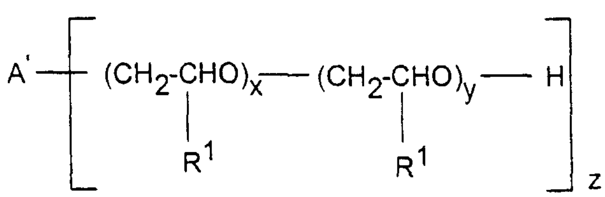

(III)

worin
x und y unabhängig voneinander eine Zahl von 0 bis 120, vorzugsweise von 5 bis 80 ist, und wobei die Summe aus x und y mindestens 5 beträgt,

z 1, 2, 3 oder 4, vorzugsweise 4 ist,
$R^1$ Wasserstoff oder Methyl bedeutet,
A' je nach $z^-$ einen Rest der Formeln (IV) bis (VII), vorzugsweise (VII)

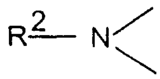
(IV)

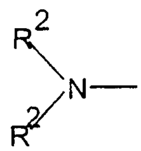
(V)

z = 2

z = 1

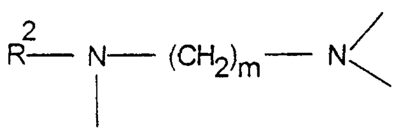
(VI)

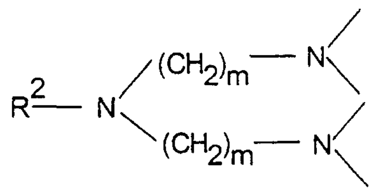
(VII)

z = 3

z = 4

bedeutet,
wobei

$R^2$ ein $C_6$-$C_{22}$-Alkyl, vorzugsweise ein $C_6$-$C_{18}$-Alkyl, und
m 2, 3 oder 4, vorzugsweise 2 oder 3 ist.

Besonders gut geeignet sind Mischungen, worin Komponente A ein Isononylphenolformaldehydharz oder ein Isononyl-/Isobutylphenolformaldehydharz und die Komponente B ein oxalkyliertes Sternamin der Formel (III) darstellt, worin

A' einen Rest der Formel (VII),
z 4,
$R^2$ $C_{12}$-$C_{14}$-Alkyl,
m 3 und

x und y unabhängig voneinander eine Zahl von 0 bis 60 bedeuten, wobei die Summe aus x und y mindestens 5 beträgt.

Ein weiterer Gegenstand der Erfindung sind Rohöle und davon abgeleitete Produkte, enthaltend als Asphalten-Dispergator eine synergistische Kombination von Alkylphenolharzen mit oxalkylierten Aminen, wie vorstehend beschrieben.

Von Rohölen abgeleitete Produkte sind beispielsweise schweres Heizöl, Schiffsöl oder Bitumen.

Die oxalkylierten Fettamine und Fettaminderivate der Formel (III) werden nach üblichen Oxalkylierungsverfahren hergestellt, indem ein Amin der Formel (IV) bis (VII) mit Ethylenoxid allein ($R^1$ ist H und der Polyoxyalkylenrest besteht aus Ethylenoxideinheiten) oder mit Propylenoxid allein ($R^1$ ist $CH_3$ und der Polyoxalkylenrest besteht aus Propylenoxideinheiten) oder mit Ethylenoxid und Propylenoxid gleichzeitig oder nacheinander ($R^1$ ist H und $CH_3$ und der Polyoxalkylenrest besteht aus Ethylenoxid- und Propylenoxideinheiten, die statistisch verteilt beziehungsweise blockweise vorliegen) umgesetzt wird. Die Umsetzung wird im allgemeinen bei einer Temperatur von 100 bis 180°C,

gegebenenfalls in Gegenwart eines alkalischen oder sauren Oxalkylierungskatalysators unter Luftausschluß durchgeführt.

Die Aminverbindungen der Formel (III) und ihre Herstellung sind in der eingangs genannten US 5 421 993 ausführlich beschrieben, die hier ausdrücklich miteinbezogen wird. Sie werden durch Oxalkylierung von Aminen der angegebenen Formeln (IV) bis (VII) zuerst mit Ethylenoxid und dann mit Propylenoxid unter Zusatz von Basen wie Alkalimetallhydroxiden erhalten. Die Umsetzung erfolgt in Stufen bei einer Temperatur von vorzugsweise 100 bis 160°C. Die Menge an eingesetztem Katalysator/Base liegt im allgemeinen bei 0,5 bis 3,0 Gew.-%, bezogen auf das eingesetzte Ausgangsamin. Die Molmenge an Ethylenoxid und Propylenoxid pro Mol Ausgangsamin entspricht den angegebenen Werten für x und y. Im einzelnen wird auf die genannte US 5 421 993 hingewiesen.

Die Verbindung der Formel (I) oder (II) kann mit NaOH, KOH, $NH_3$ oder Amin ganz oder teilweise neutralisiert werden. Sowohl saure als auch neutralisierte Formen sind wirksam.

Für manche Öle ist ein sauer eingestellter Asphaltendispergator vorteilhaft. Dies kann durch Zugabe von Säuren wie z.B. Essigsäure erreicht werden. Geeignet sind besonders organische Säuren mit Tensideigenschaften, wie Mono- oder Dialkylbenzolsulfonsäuren, Petrolsulfonsäuren und Alkansulfonsäuren.

Die erfindungsgemäße synergistische Mischung wird in einer Konzentration von 0,5 bis 10.000 ppm, vorzugsweise von 2 bis 2.000 ppm eingesetzt.

Zur leichteren Dosierung kann diese Mischung als Lösung in einem ölmischbaren Lösemittel formuliert werden, wie beispielsweise aromatische Kohlenwasserstoffe oder Mischungen von Kohlenwasserstoffen und einem aliphatischen Alkohol.

Da die erfindungsgemäße synergistische Mischung auf einer Kombination von Substanzen basiert, kann sie auf eine Änderung der Zusammensetzung des Öls weniger empfindlich sein; dies verbessert ihre Zuverlässigkeit.

Beispiele

Geprüfte Substanzen

Komponente A:

A I: Isononylphenolformaldehydharz (sauer katalysiert), mit KOH neutralisiert

A II: Isononylphenolformaldehydharz (sauer katalysiert), nicht neutralisiert

A III: Alkylphenolformaldehydharz (sauer katalysiert), mit KOH neutralisiert, Alkylkette = 1:1 Isononyl-Isobutyl-Mischung

Der durchschnittliche Wert für n, berechnet aus dem Molekulargewicht, betrug etwa 8,6.

Komponente B:

B I: Oxalkyliertes Sternamin

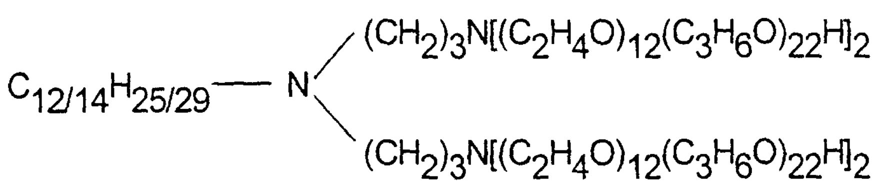

B II: Oxalkyliertes Sternamin

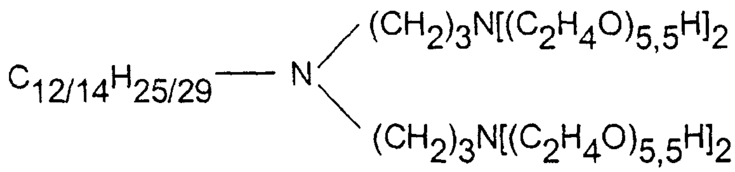

B III: Oxalkyliertes Sternamin

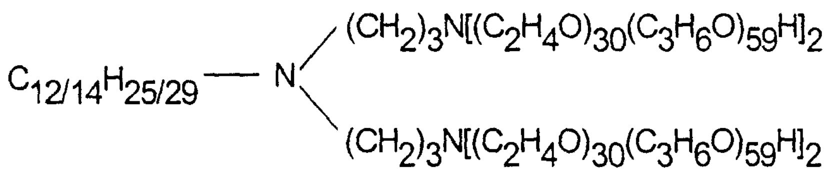

Zur Herstellung des Formaldehyd-Harzes A I wurde p-Nonylphenol in Gegenwart katalytischer Mengen Alkylbenzolsulfonsäure mit einer äquimolaren Menge einer 35 Gew.-%igen Formalinlösung zur Reaktion gebracht, das Reaktionsgemisch durch Auskreisen mit einer Mischung höher siedender aromatischer Kohlenwasserstoffe (Siedebereich 185-215°C) vom Wasser befreit und mit Kaliumhydroxid neutralisiert. Das rotbraune Harz wurde in ®Solvent Naphtha auf einen Feststoffgehalt von 50 % verdünnt. Das gelchromatographisch bestimmte Molekulargewicht (Eichung gegen Polystyrol-Standards) liegt bei 2000 g/mol.

Prüfung der Wirksamkeit von Asphaltendispergatoren

Flockungstest: Methode

Die Dispergierung, das Ausfällen von Asphaltenen hängt von der Natur des Kohlenwasserstoffmediums ab. Asphaltene sind in aromatischen, aber nicht in aliphatischen Kohlenwasserstoffen löslich. Somit können Dispergatoren getestet werden, indem man das Öl oder extrahierte Asphaltene in einem aromatischen Lösemittel bzw. Cycloalkan löst und indem man dann einen aliphatischen Kohlenwasserstoff zugibt, bis Flockung und/oder ein Niederschlag auftritt. Diese Flockung kann mittels der Lichttransmission beobachtet werden. Bei der Zugabe des aliphatischen Kohlenwasserstoffes erhöht sich zunächst die Transmission durch den Verdünnungseffekt. Wenn die Asphaltene zu flockulieren beginnen, dann reduziert die Trübung die Lichttransmission. Wie in Fig. 1 dargestellt, wird eine maximale Lichttransmission beobachtet; diese wird als Beginn der Flockung verwendet. Dieser Test wurde in verschiedenen wissenschaftlichen Publikationen beschrieben, z.B. D. Kessel, H.J. Neumann und I Rahimian, "Asphaltics in Crude Oil-water Emulsions", Proc. 1 st World Congress on Emulsions, Paris, 1993, Paper 1-22-071.

Je mehr aliphatisches Fällungsmittel zugegeben werden kann vor Eintritt der Flockung, desto besser ist das Dispergiermittel. Zum Vergleich von Dispergiermitteln kann die zum Auslösen der Flockung benötigte Menge an Ausfällmittel dienen. Um die relative Wirksamkeit P von Dispergiermitteln zu beurteilen, wurde folgende Formel benutzt:

$$P = 100\ (X/X_0\ -1)$$

Darin ist X die Menge an Fällungsmittel, die in Anwesenheit eines Dispergiermittels nötig ist, um die Flockung auszulösen. $X_0$ ist die Menge an Fällungsmittel, die die Flockung auslöst, wenn kein Dispergiermittel anwesend ist.

Prinzip des Dispergiertestes

Auch dieser Test basiert auf der Tatsache, daß Asphaltene in aromatischen, aber nicht in aliphatischen Kohlenwasserstoffen löslich sind. Somit können Dispergatoren getestet werden, indem man das Öl oder extrahierte Asphaltene in einem aromatischen Lösemittel löst und indem man dann einen aliphatischen Kohlenwasserstoff zugibt, um einen Niederschlag zu erzeugen. Da Asphaitene von dunkler Farbe sind, kann das Ausmaß des Niederschlages durch eine kolorimetrische Messung der überstehenden Flüssigkeit bestimmt werden. Je dunkler die überstehende

Flüssigkeit ist, desto mehr Asphaltene bleiben dispergiert, d.h. umso besser ist der Dispergator. Dieser Test wird beschrieben in CA 2 029 465. In unserer Version des Tests wird das Fällungsmedium so ausgewählt, daß die Asphaltene zum größten Teil, aber nicht komplett ausfallen.

Vorschrift Dispergiertest

a) Eine 25 %ige Öl-Lösung in Toluol wird filtriert, um Verunreinigungen zu beseitigen;
b) 9,5 ml Heptan als Fällungsmittel für Asphaltene und 0,5 ml Toluol/Dispergator-Mischung (25:1) in ein gut 10 ml fassendes graduiertes Glasröhrchen vorlegen und gut schütteln. Dies entspricht einer Dispergatorkonzentration von 2000 ppm. Bei Bedarf kann die Menge Dispergator variiert werden. Für die Nullproben wird reines Toluol verwendet;
c) in das Glasröhrchen wird dann 0,1 ml von der gefilterten Öl-Lösung dazugegeben und ebenfalls gut geschüttelt;
d) das Ganze 2 Stunden ohne Erschütterungen stehenlassen. Die ausgefällten Asphaltene sollen sich am Boden des Röhrchens sammeln können;
e) nach Ablauf dieser Zeit wird das Volumen des Sediments an Hand der Graduierung abgeschätzt, das Aussehen der gesamten Probe protokolliert und dann wird von der überständigen Phase 1 ml vorsichtig mit einer Pipette aufgenommen;
f) die abgesaugte Menge wird in 5 ml einer 99:1 Toluol-Triethanolamin-Mischung gelöst und bei 600 nm photometriert.

Bewertung des Dispergiertests

Als relatives Maß für die Dispergierung wird folgender Ausdruck genommen

$$A = 100\,(D-D_0)/D_0,$$

wobei D und $D_0$ optische Dichte von Meßlösung und Blindprobe sind. Der maximal erreichbare Wert von A, $A_{max}$, entspricht vollständiger Dispergierung der Asphaltene. Sie kann abgeschätzt werden, indem ein Versuch ohne Dispergator, mit Toluol anstatt Heptan, durchgeführt wird - dadurch bleiben die Asphaltene vollständig dispergiert.

Das Volumen des Sediments liefert eine weitere Information über die Wirksamkeit des Dispergators. Je kleiner die Menge an Sediment ist, desto besser dispergiert ist die Substanz.

Beispiel 1:

Substanzen A I, B I und eine Mischung von A I und B I wurden an einem leichten Rohöl aus der Nordsee mit dem Flokkulationstest geprüft. Das Öl wurde 1:1 mit Cyclohexan gemischt und n-Pentan schrittweise zugegeben. Die Dispergatordosis betrug 2000 ppm.

Die Ergebnisse in Tabelle 1 zeigen, daß die Mischung von I und II besser als die Einzelkomponenten wirkt.

Tabelle 1

| Dispergator | Rel. Dispergierwirkung P[%] | |
|---|---|---|
| 1:1 Mischung von A I und B I | 14,5 | erfindungsgemäße Mischung |
| A I | 3,9 | Vergleichsversuch |
| B I | 1,2 | Vergleichsversuch |

Beispiel 2

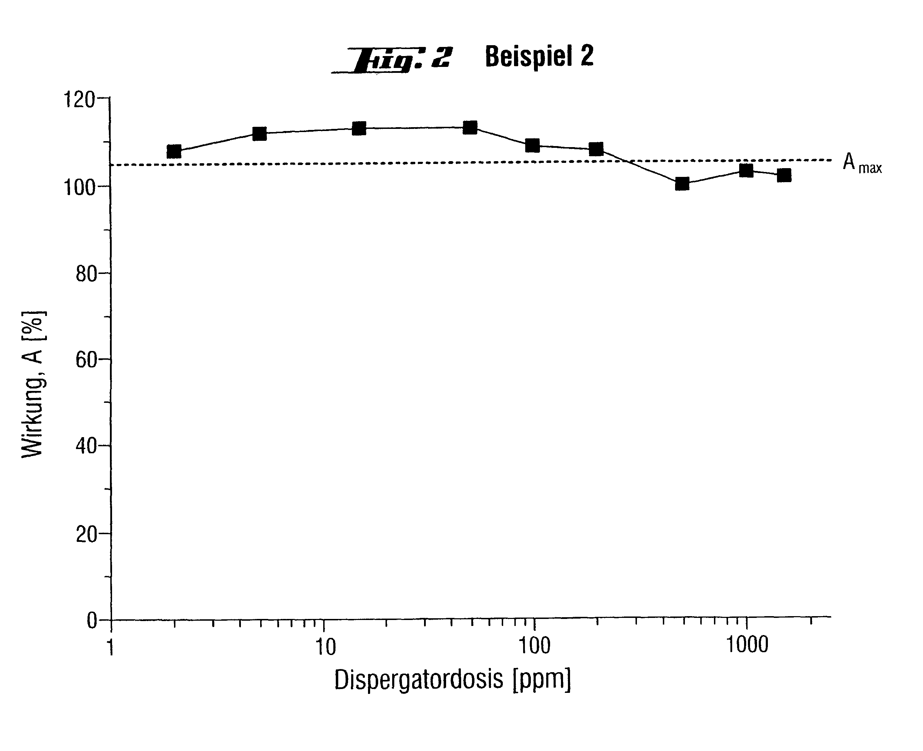

Mit einem asphaltenreichen Öl aus Venezuela wurde eine erfindungsgemäße Mischung mit dem Dispergiertest geprüft. Der Dispergator bestand aus einer Mischung von 7 Teilen Substanz A I und 3 Teilen Substanz B II. Die Dispergierwirkung in Abhängigkeit von der Dosis wird in Fig. 2 gezeigt. Diese Mischung zeigt schon bei einer Dosis von 2 ppm eine gute Dispergierwirkung. Bezogen auf die Menge des Öls entspricht diese Dosis 500 ppm. In der Praxis wird die erforderliche Dosis sowohl von der Art des Öls als auch von den anwendungstechnischen Anforderungen

abhängen.

Beispiele 3 - 7

Mit dem in Beispiel 2 erwähnten asphaltenreichen Öl aus Venezuela wurden erfindungsgemäße Mischungen mit dem Dispergiertest geprüft. Die Dosis betrug 2000 ppm.

| Beispiel Nr. | Komponente A | Komponente B | Dispergierwirkung A [%] | Sedimentvolumen ml |
|---|---|---|---|---|
| 3 | A I 20% | B I 80% | 121 | 0 |
| 4 | A II 50% | B I 50% | 123 | 0 |
| 5 | A II 50% | B I 50% | 115 | 0 |
| 6 | A I 50% | B II 50% | 125 | Spur |
| 7 | A I 50% | B III 50% | 121 | 0 |
| Nullprobe | ----- | ----- | 0 | 0,45 |

Bei dieser Versuchsreiche betrug die maximale Dispergierwirkung $A_{max}$ ca. 120 %.

**Patentansprüche**

**1.** Synergistische Mischung enthaltend als Asphalten-Dispergator 5 bis 95 Gew.-% einer Verbindung A der Formeln (I) oder (II)

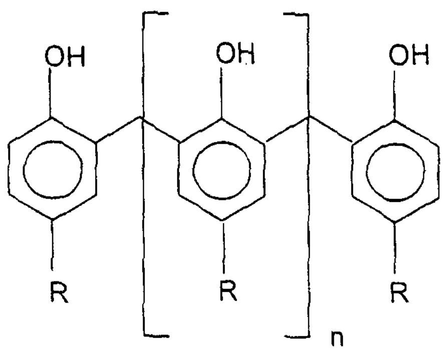

(I)

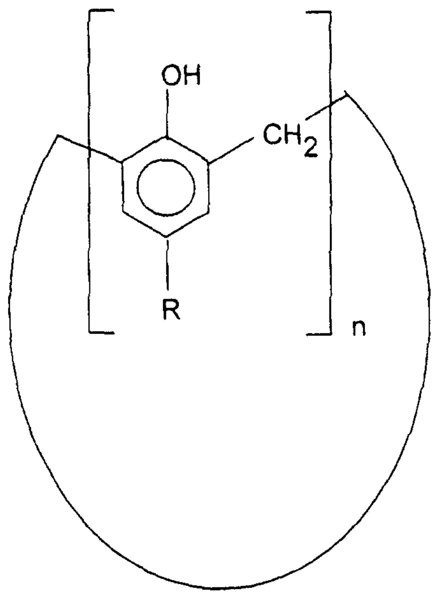

(II)

wobei

n 2 bis 12 und
R $C_3$-$C_{24}$-Alkyl, $C_6$-$C_{12}$-Aryl oder -Hydroxyaryl bzw. $C_7$-$C_{12}$-Aralkyl bedeuten,

und
5 bis 95 Gew.-% einer Verbindung B der Formel (III)

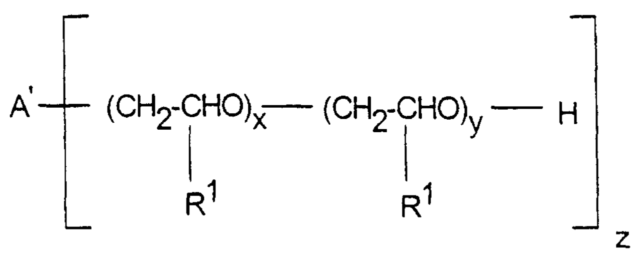

(III)

worin

$R^1$ Wasserstoff oder Methyl bedeutet,
z 1, 2, 3 oder 4 ist,

x und y unabhängig voneinander eine Zahl von 0 bis 120 ist, und wobei die Summe von x und y mindestens 5 beträgt,

A' je nach z- einen Rest der Formeln (IV) bis (VII) bedeutet

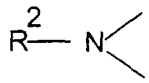 (IV)

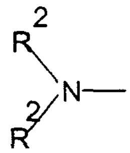 (V)

z = 2

z= 1

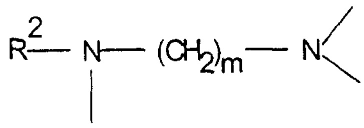 (VI)

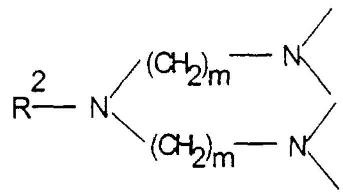 (VII)

z = 3

z = 4

wobei

$R^2$ ein $C_6$-$C_{22}$-Alkylrest und
m 2, 3 oder 4 ist.

**2.** Synergistische Mischung gemäß Anspruch 1, enthaltend als Asphalten-Dispergator 20 bis 80 Gew.-% einer Verbindung A der Formeln (I) oder (II), worin

n 5 bis 9 und
R $C_4$-$C_{12}$-Alkyl bedeuten,

und
20 bis 80 Gew.-% einer Verbindung B der Formel (III), worin

$R^1$ einen $C_6$-$C_{18}$-Alkylrest,
z 4,

x und y unabhängig voneinander eine Zahl von 5 bis 80,

A' einen Rest der Formel (VII),
m 2 oder 3, und
$R^2$ Wasserstoff oder Methyl bedeuten.

**3.** Synergistische Mischung gemäß Anspruch 1, **dadurch gekennzeichnet, daß** es sich bei der Komponente A um Isononylphenolformaldehydharz oder Isononyl-/Isobutylphenolformaldehydharz und bei der Komponente B um ein oxalkyliertes Sternamin der Formel (III) handelt, worin

A' einen Rest der Formel (VII),
z 4,
$R^2$ $C_{12}$-$C_{14}$-Alkyl,
m 3 und

x und y unabhängig voneinander eine Zahl von 0 bis 60 bedeuten, wobei die Summe aus x und y mindestens 5 beträgt.

**4.** Verwendung einer synergistischen Mischung gemäß mindestens einem der Ansprüche 1 bis 3 als Asphalten-Dispergator in Rohölen und davon abgeleiteten Produkten.

**5.** Rohöle und davon abgeleitete Produkte, enthaltend Asphaltene und, als Asphalten-Dispergator, eine synergistische Mischung gemäß mindestens einem der Ansprüche 1 bis 3.

**6.** Verfahren zum Dispergieren von Asphaltenen in Rohölen und davon abgeleiteten Produkten, **dadurch gekennzeichnet, daß** als Dispergator eine synergistische Mischung gemäß mindestens einem der Ansprüche 1 bis 3 in einer Menge von 0,5 bis 10.000, vorzugsweise von 2 bis 2.000 ppm zugegeben wird.

**7.** Verfahren gemäß Anspruch 6, **dadurch gekennzeichnet, daß** zusätzlich Mono- oder Dialkylbenzolsulfonsäuren, Petrolsulfonsäuren, Alkansulfonsäuren oder beliebige Mischungen daraus verwendet werden.

## Claims

**1.** A synergistic mixture comprising, as asphaltene dispersant,
from 5 to 95% by weight of a compound A of the. formula (I) or (II)

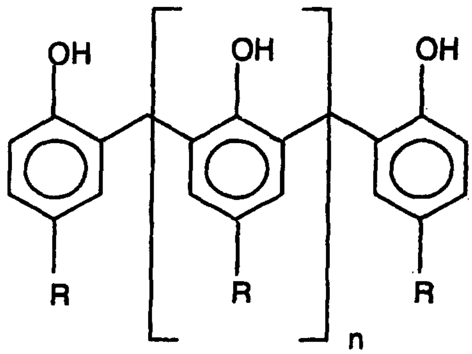

(I)

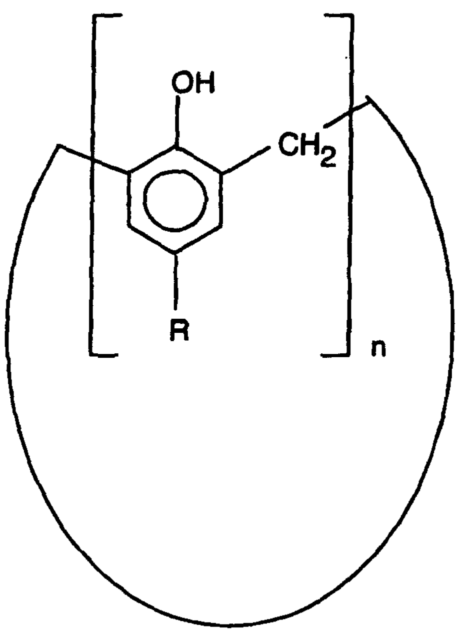

(II)

where

n is from 2 to 12 and
R is $C_3$-$C_{24}$-alkyl, $C_6$-$C_{12}$-aryl or -hydroxyaryl or $C_7$-$C_{12}$-aralkyl,

and
from 5 to 95% by weight of a compound B of the formula (III)

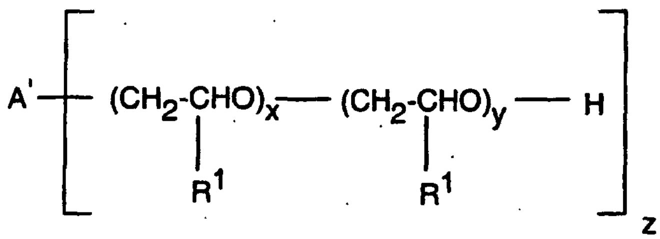

(III)

in which

$R^1$ is hydrogen or methyl,
z is 1, 2, 3 or 4,

x and y, independently of one another, are a number from 0 to 120, the sum x + y being at least 5,

A' is, depending on z, a radical of the formulae (IV) to (VII)

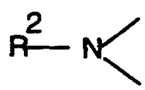

(IV)

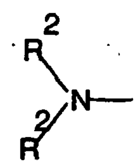

(V)

z=2

z=1

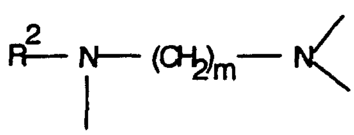

(VI)

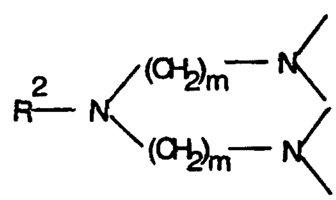

(VII)

z=3

z=4

where

$R^2$ is a $C_6$-$C_{22}$-alkyl radical and
m is 2, 3 or 4.

**2.** The synergistic mixture as claimed in claim 1, comprising, as asphaltene dispersant, from 20 to 80% by weight of a compound A of the formula (I) or (II) in which

n is from 5 to 9 and
R is $C_4$-$C_{12}$-alkyl,

and
from 20 to 80% by weight of a compound B of the formula (III) in which

$R^1$ is a $C_6$-$C_{18}$-alkyl radical,
z is 4,

x and y, independently of one another, are a number from 5 to 80,

A' is a radical of the formula (VII),
m is 2 or 3, and
$R^2$ is hydrogen or methyl.

**3.** The synergistic mixture as claimed in claim 1, wherein component A is isononylphenol-formaldehyde resin or isononyl/isobutylphenol-formaldehyde resin and component B is an oxalkylated star-shaped amine of the formula (III) in which

A' is a radical of the formula (VII),
z is 4,
$R^2$ is $C_{12}$-$C_{14}$-alkyl,
m is 3 and

x and y, independently of one another, are a number from 0 to 60, the sum x + y being at least 5.

**4.** The use of a synergistic mixture as claimed in at least one of claims 1 to 3 as asphaltene dispersant in crude oils and products derived therefrom.

**5.** A crude oil or product derived therefrom comprising asphaltenes and, as asphaltene dispersant, a synergistic mixture as claimed in at least one of claims 1 to 3.

**6.** A process for dispersing asphaltenes in crude oils and products derived therefrom, which comprises adding, as dispersant, a synergistic mixture as claimed in at least one of claims 1 to 3 in an amount of from 0.5 to 10,000 ppm, preferably from 2 to 2000 ppm.

**7.** The process as claimed in claim 6, which comprises additionally using mono- or dialkylbenzenesulfonic acids, petroleum sulfonic acids, alkanesulfonic acids or any desired mixtures thereof.

## Revendications

**1.** Mélange synergique contenant en tant que dispersant d'asphaltènes
de 4 à 95 % en masse d'un composé A de formules (I) ou (II)

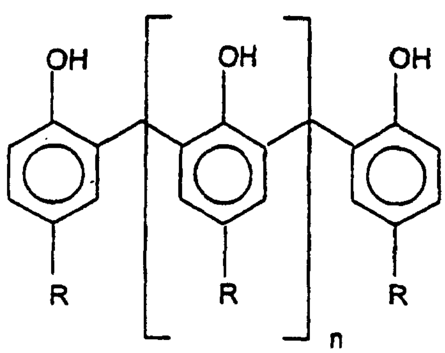

(I)

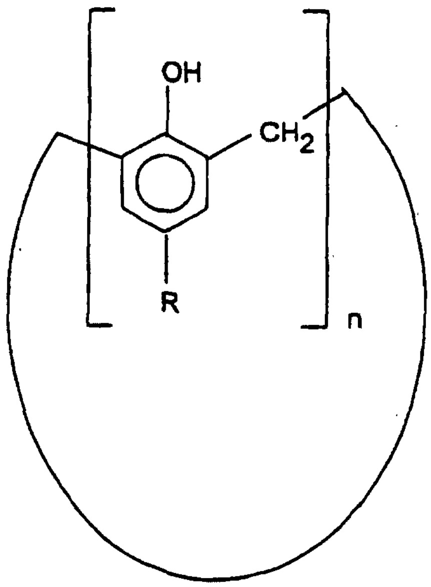

(II)

où

n va de 2 à 12 et
R représente des groupes alkyle en $C_3$-$C_{24}$, aryle en $C_6$-$C_{12}$ ou hydroxyaryle en $C_6$-$C_{12}$ ou aralkyle en $C_7$-$C_{12}$,

et
de 5 à 95 % en masse d'un composé B formule (III)

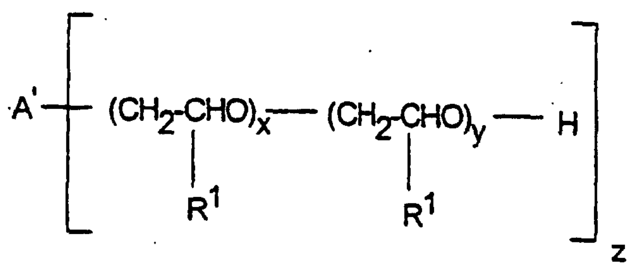

(III),

où

$R^1$ représente un atome d'hydrogène ou un groupe méthyle,

z vaut 1, 2, 3 ou 4,

x et y sont, indépendamment l'un de l'autre, un entier de 0 à 120, et la somme de x et de y étant d'au moins 5,

A' à chaque fois en fonction de z, représente un reste de formules (IV) à (VII)

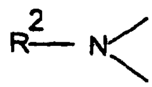 (IV)

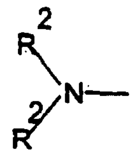 (V)

z=2

z=1

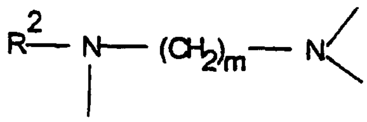 (VI)

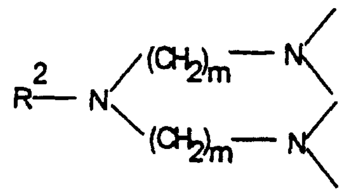 (VII)

z=3

z=4

où

$R^2$ représentant un reste alkyle en $C_6$-$C_{22}$ et

m valant 2, 3 ou 4.

**2.** Mélange synergique selon la revendication 1, contenant en tant que dispersant d'asphaltènes de 20 à 80 % en masse d'un composé A de formules (I) ou (II), où

n va de 5 à 9 et
R représente un reste alkyle en $C_4$-$C_{12}$,

et
de 20 à 80 % en masse d'un composé B de formule (III), où

$R^1$ représente un reste alkyle en $C_6$-$C_{18}$,
z vaut 4,

x et y représentent, indépendamment l'un de l'autre, un nombre de 5 à 80,

$A^1$ représente un reste de formule (VII),
m vaut 2 ou 3, et
$R^2$ représente un âtome d'hydrogène ou un groupe méthyle.

**3.** Mélange synergique selon la revendication 1, **caractérisé en** de qu'il s'agit pour le composant A d'une résine isononylphénolformaldéhyde ou d'une résine isononyl-/isobutylphénolformaldéhyde et pour le composant B, d'une amine étoilée alkoxylée de formule (III), où

$A^1$ représente un reste de for mule (VII),
z vaut 4,
$R^2$ représente un groupe alkyle en $C_{12}$-$C_{14}$,
m vaut 3 et

x et y représentent, indépendamment l'un de l'autre, un nombre de 0 à 60, la somme de x et de y étant d'au moins 5.

**4.** Utilisation d'un mélange synergique selon au moins l'une des revendications 1 à 3 en tant que dispersant d'asphaltènes dans des pétroles bruts et leurs produits dérivés.

**5.** Pétroles bruts et leurs produits dérivés contenant des asphaltènes et, en tant que dispersant d'asphaltènes, un mélange synergique selon au moins l'une des revendications 1 à 3.

**6.** Procédé pour disperser des asphaltènes dans des pétroles bruts et leurs produits dérivés, **caractérisé en ce qu'**on utilise, en tant que dispersant, un mélange synergique selon au moins l'une des revendications 1 à 3, en une quantité de 0,5 à 10 000, de préférence de 2 à 2 000 ppm.

**7.** Procédé selon la revendication 6, **caractérisé en ce qu'**on utilise des plus des acides mono- ou dialkylbenzènesulfoniques, des acides persulfoniques, des acides alcanesulfoniques ou des mélanges arbitraires de ces acides.

Fig. 1 Flockungstest: Prinzip

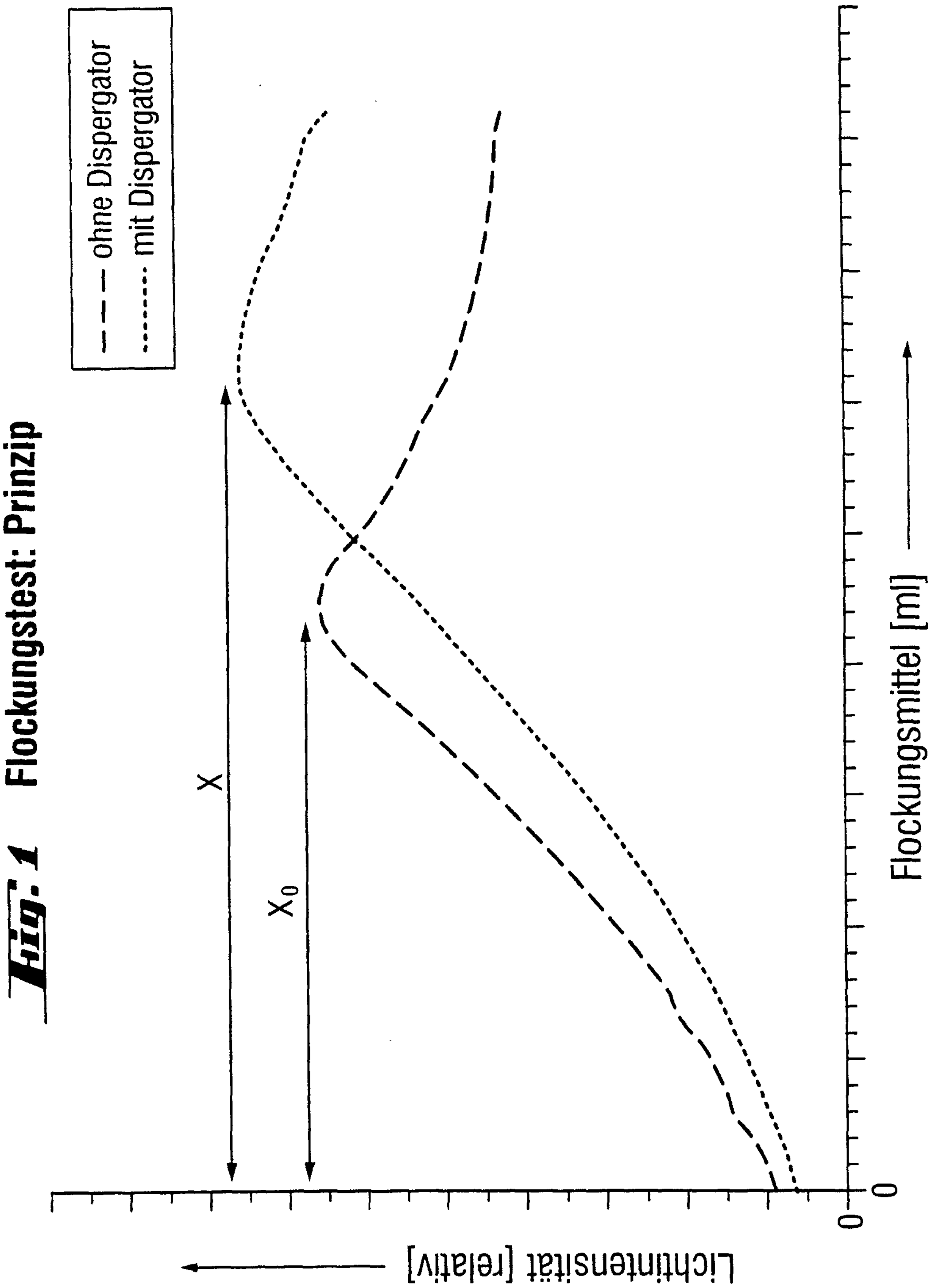

Fig. 2 Beispiel 2